# EUROPEAN PATENT APPLICATION

(11) **EP 4 464 307 A1**
(43) Date of publication of application: **20.11.2024**
(21) Application number: 24175095.9
(22) Date of filing: 10.05.2024
(51) Int. Cl.: A61K 8/25, A61K 8/26, A61K 8/34, A61K 8/73, A61Q 1/10

(54) **COSMETIC COMPOSITION AND METHODS OF MAKING THE SAME**

(30) Priority: 10.05.2023 US 202363465277 P; 01.05.2024 US 202418652024
(71) Applicant: MPLUS COSMETICS S.R.L., 20065 Milano (IT)
(72) Inventor: Kirienko, Alexandra, 20065 Milano (IT); Robles, Dagna Sheylia Garcia, 20065 Milano (IT)
(74) Representative: Berggren Oy

(57) **Abstract**

A method of making a cosmetic composition which includes providing a solvent, at least one gelling agent, at least one dispersant, at least one salt, at least one pH adjuster, and at least one preservative in a mixer to provide a first combination, homogenizing the first combination to provide a first homogenous mixture, performing a first pH monitoring step to determine if a pH of the first homogenous mixture is within a first specified pH range, combining the first homogenous mixture with at least one colorant to provide a second combination, performing a second pH monitoring step to determine if a pH of the second combination is within a second specified pH range, homogenizing the second combination to provide a second homogenous mixture, and unloading the second homogenous mixture from the mixer.

## Description

### CROSS-REFERENCE TO RELATED APPLICATION

This application claims priority to U.S. Provisional Application No. 63/465,277, filed on May 10, 2023 and U.S. Application No. 18/652024, filed on May 1, 2024, the contents of each of which are expressly incorporated herein in their entirety.

### TECHNICAL FIELD

The present disclosure is directed to cosmetic compositions and methods of making the same.

### BACKGROUND

There is a need in the art for a water-based cosmetic composition that provides all-over, long wear without the transfer of natural gums. In addition, there is a need in the art for an alternative to menthol, which provides a cooling effect to the skin but can often cause irritation.

### SUMMARY

The present disclosure is directed to a stable cosmetic composition. The cosmetic composition of the present disclosure is configured to color a surface, particularly the skin. According to some aspects, the cosmetic composition includes one or more of each of a gelling agent, a salt, a colorant, a pH adjuster, a dispersant, a preservative, and a solvent.

Also disclosed are methods of making the cosmetic composition as described herein. The method may including providing a solvent, at least one gelling agent, at least one dispersant, at least one salt, at least one pH adjuster, and at least one preservative in a mixer to provide a first combination, homogenizing the first combination to provide a first homogeneous mixture, performing a first pH monitoring step to determine if a pH of the first homogeneous mixture is within a first specified pH range, optionally modifying the pH of the first homogeneous mixture to be within the first specified pH range, combining the first homogenous mixture with at least one colorant to provide a second combination, performing a second pH monitoring step to determine if a pH of the second combination is within a second specified pH range, optionally modifying the pH of the second combination to be within the second specified pH range, homogenizing the second combination to provide a second homogenous mixture, and unloading the second homogenous mixture from the mixer at an elevated temperature to provide the cosmetic composition.

### BRIEF DESCRIPTION OF THE DRAWINGS

FIG. 1 shows an example process according to the present disclosure.
FIG. 2 shows an example process according to the present disclosure.
FIG. 3 shows an example process according to the present disclosure.
FIG. 4 shows example components of the phases shown in FIGs. 1-3.

### DETAILED DESCRIPTION

The present disclosure is directed to a stable cosmetic composition. The cosmetic composition of the present disclosure is configured to color a surface, particularly the skin. According to some aspects, the cosmetic composition includes one or more of each of a gelling agent, a salt, a colorant, a pH adjuster, a dispersant, a preservative, and a solvent. According to some aspects, the cosmetic composition includes at least one component that provides a cooling effect without requiring menthol such that the cosmetic composition is menthol-free. The cosmetic composition may further provide a bold, glide-able color on a surface, such as on the skin, that is long lasting and provides minimal transfer upon contact with a second surface. Additionally or alternatively, the cosmetic composition may provide a powdery finish without requiring a wax. Also disclosed are methods of making the cosmetic composition as described herein.

According to some aspects, the cosmetic composition may include one or more gelling agents. As used herein, the term "gelling agent" refers to a component that forms a gel in an aqueous phase. According to some aspects, each of the one or more gelling agents may individually be hydrophilic or lipophilic. In some non-limiting examples, one or more of the gelling agents may be natural or naturally originated. As used herein, "naturally originated" refers to a component provided by modifying a natural gelling agents.

Example gelling agents useful according to the present disclosure include natural extracts. Non-limiting examples of natural extracts include extracts of algae, such as a *Chondrus crispus* extract. It should be understood that a *Chondrus crispus* extract may include more than one component, including flavonoids, polyphenols, tannins, and/or polysaccharides such as carrageenan. Additionally or alternatively, the one or more gelling agents may include carrageenan from a source other than *Chondrus crispus* and/or may include purified carrageenan. According to some aspects, the gelling agent of the present disclosure may be sufficient to provide a cooling effect on the skin without requiring menthol. In this way, the cosmetic composition may be menthol-free.

According to some aspects, the cosmetic composition may include each gelling agent individually at a concentration between about 0.01 and 20% w/v, optionally between about 0.01 and 15% w/v, and optionally between about 0.1 and 10% w/v, including any value therebetween. According to some aspects, the cosmetic composition may include a total gelling agent concentration between about 0.01 and 20% w/v, optionally between about 0.01 and 15% w/v, and optionally between about 0.1 and 10% w/v, including any value therebetween.

According to some aspects, the cosmetic composition may include one or more salts. According to some aspects, each of the one or more salts may individually be an inorganic salt. Non-limiting examples of inorganic salts include phosphate, sulphate, sulphite, bromide, carbonates, chloride, chlorate, fluoride, iodate, iodide, nitrate, molybdate, selenite, oxide, silicate, and gluconate salts. According to some aspects, the cosmetic composition may include sodium chloride, sodium gluconate, or a combination thereof. Additionally or alternatively, each of the one or more salts may individually be an organic salt.

In some non-limiting examples, the one or more salts include acetyl tetrapeptide-2 acetate, alanine glutamate, allantoin acetyl methionine, allantoin ascorbate, allantoin biotin, allantoin calcium pantothenate, allantoin galacturonic acid, allantoin paba, allantoin polygalacturonic acid, aluminum acetate, aluminum acetate solution, aluminum acetylacetonate, aluminum benzoate, aluminum bromohydrate, aluminum butoxide, aluminum chloride, aluminum chlorohydrate, aluminum chlorohydrex, aluminum chlorohydrex Peg, aluminum chlorohydrex PG, aluminum citrate, aluminum diacetate, aluminum dibenzoate/Stearate hydroxide, aluminum dicetyl phosphate, aluminum dichlorohydrate, aluminum dichlorohydrex Peg, aluminum dichlorohydrex PG, aluminum fluoride, aluminum isopropoxide, aluminum lactate, aluminum methionate, aluminum PCA, aluminum sesquichlorohydrate, aluminum sesquichlorohydrex Peg, aluminum sesquichlorohydrex PG, aluminum silicate, aluminum sucrose octasulfate, aluminum sulfate, aluminum triformate, aluminum tris-hydroxynitrosoaniline, aluminum zirconium octachlorohydrate, aluminum zirconium octachlorohydrex gly, aluminum zirconium pentachlorohydrate, aluminum zirconium pentachlorohydrex gly, aluminum zirconium tetrachlorohydrate, aluminum zirconium tetrachlorohydrex gly, aluminum zirconium tetrachlorohydrex Peg, aluminum zirconium tetrachlorohydrex PG, aluminum zirconium trichlorohydrate, aluminum zirconium trichlorohydrex gly, aluminum/Magnesium azelate hydroxide, aluminum/Magnesium hydroxide stearate, ammonium acetate, ammonium acrylate, ammonium alum, ammonium benzoate, ammonium bicarbonate, ammonium bisulfite, ammonium C6-16 perfluoroalkylethyl phosphate, ammonium carbamate, ammonium carbonate, ammonium caseinate, ammonium chloride, ammonium ferric pentetate, ammonium fluoride, ammonium fluorosilicate, ammonium glycolate, ammonium glycyrrhizate, ammonium hexafluorophosphate, ammonium iodide, ammonium lactate, ammonium laureth-6 carboxylate, ammonium laureth-8 carboxylate, ammonium methacrylate, ammonium molybdate, ammonium monofluorophosphate, ammonium nitrate, ammonium persulfate, ammonium phosphate, ammonium phosphatidyl rapeseedate, ammonium propionate, ammonium shellacate, ammonium sulfate, ammonium sulfite, ammonium thiocyanate, ammonium thioglycolate, ammonium thiolactate, ammonium trichloroacetate, ammonium vanadate, ammonium xanthommatin, amodimethicone hydroxystearate, antimony potassium tartrate, arginine aspartate, arginine bicarbonate, arginine ferulate, arginine glutamate, arginine hexyldecyl phosphate, arginine mandelate, arginine PCA, barium chloride, barium gluconate, barium sulfate, barium sulfide, bis(Tripeptide-1) Copper acetate, bis-diaminopyrimidine oxide zinc acetate, bismuth citrate, bismuth oxychloride, bismuth subgallate, bismuth subnitrate, bittern, boron nitride, brucine sulfate, caffeine salicylate, calcium acetate, calcium acrylate, calcium ascorbate, calcium aspartate, calcium benzoate, calcium carbonate, calcium carboxymethyl cellulose, calcium carnaubate, calcium chitosan, calcium chloride, calcium citrate, calcium cyclamate, calcium dihydrogen phosphate, calcium disodium edta, calcium fluoride, calcium fructoborate, calcium fructoheptonate, calcium glucoheptonate, calcium gluconate, calcium glycerophosphate, calcium hydroxymethionine, calcium hydroxymethylbutyrate, calcium hypochlorite, calcium ketogluconate, calcium lactate, calcium magnesium silicate, calcium monofluorophosphate, calcium pantetheine sulfonate, calcium pantothenate, calcium paraben, calcium PCA, calcium phosphate, calcium phosphoryl oligosaccharides, calcium polyoxymethylene pyrrolidone, calcium potassium carbomer, calcium potassium sodium phosphate, calcium propionate, calcium pyrophosphate, calcium saccharin, calcium salicylate, calcium silicate, calcium sorbate, calcium starch isododecenylsuccinate, calcium stearoyl lactylate, calcium sulfate, calcium sulfate hydrate, calcium sulfide, calcium tartrate, calcium thioglycolate, calcium thioglycolate hydroxide, calcium titanate, calcium trifluoroacetate, chitosan adipate, chitosan ascorbate, chitosan glycolate, chitosan salicylate, chloramine t, chlorhexidine diacetate, chlorhexidine digluconate, chlorhexidine dihydrochloride, chlorhexidine diundecylenate, chlorophyllin-copper complex, chlorophyllin-iron complex, ciclopirox olamine, citrate buffer, cobalt chloride, cobalt gluconate, cobamamide, copper adenosine triphosphate, copper aminoacetylamino imidazolyl propanoate, copper ascorbyl phosphate succinoyl tripeptide-34, copper carbonate hydroxide, copper chlorophyll, copper citrate, copper gluconate, copper heptapeptide-14 pantothenate, copper palmitoyl heptapeptide-14, copper PCA, copper PCA methylsilanol, copper picolinate, copper sulfate, copper usnate, cupric acetate, cupric chloride, cysteamine hcl, cysteamine thioglycolate, dea-cetyl phosphate, dea-hydrolyzed lecithin, dea-methoxycinnamate, diammonium citrate, diammonium dithiodiglycolate, diammonium ferric pentetate, diammonium phosphate, dibehenamidopropyldimethylamine dilinoleate, diglycol guanidine succinate, dihydroxyethyl tallowamine oleate, dilithium oxalate, dimethicone propylethylenediamine behenate, dimethylaminoethanol tartrate, dipotassium azelate, dipotassium bryonolyl succinate, dipotassium glycyrrhizate, dipotassium oxalate, dipotassium phosphate, disodium adenosine phosphate, disodium adenosine triphosphate, disodium aluminate glucarate/Gluconate/Glycolate/Tartrate/Oxalate, disodium ascorbyl sulfate, disodium azelate, disodium carboxyethyl siliconate, disodium cocaminopropyl iminodiacetate, disodium coco-glucoside citrate, disodium coco-glucoside sulfosuccinate, disodium cocoyl glutamate, disodium cupric citrate, disodium dicarboxyethyl cocopropylenediamine, disodium dodecenylsuccinate, disodium fumarate, disodium glucarate/Gluconate/Glucuronate/Glycolate/Ketogluconate/Oxalate/Tartrate/Tartronate, disodium glycyrrhizate, disodium hydroxyethyliminodiacetate, disodium hydroxysulfinoacetate, disodium hydroxysulfoacetate, disodium isostearyl ascorbyl phosphate, disodium itaconate, disodium laurylglucosides hydroxypropyl citrate, disodium phenylene bis-isostearylphosphate, disodium phosphate, disodium phosphocreatine, disodium ppg-2-isodeceth-7 carboxyamphodiacetate, disodium pyrophosphate, disodium rutinyl disulfate, disodium sebacate, disodium sebacate/Palm kernelate triglyceride, disodium selenite, disodium succinate, disodium succinoyl farnesylcysteine, disodium succinoyl glycyrrhetinate, disodium sulfomethyl cocosuccinate, disodium tartrate, disodium tetrapropenyl succinate, disodium ubiquinone, disoyamidoethyl hydroxyethyl ammonium lactate, dmem, ethanolamine dithiodiglycolate, ethanolamine glycerophosphate, ethanolamine thioglycolate, Ethyl hydroxy picolinium lactate, Ethyl lauroyl arginate hcl, ferric ammonium citrate, ferric ammonium ferrocyanide, ferric chloride, ferric citrate, ferric ferrocyanide, ferric glycerophosphate, ferric nitrate, ferric sulfate, ferrous ammonium sulfate, ferrous aspartate, ferrous fumarate, ferrous glucoheptonate, ferrous gluconate, ferrous lactate, ferrous sulfate, germanium citrate, germanium lactate, glucosamine salicylate, guanidine carbonate, guanidine hcl, guanidine phosphate, heptapeptide-50 trifluoroacetate, heptasodium hexacarboxymethyl dipeptide-12, hexamidine paraben, homarine hcl, hydrolyzed hyaluronic acid, hydroxyethyl dimethylammonium lactate, hydroxylamine hcl, hydroxylamine sulfate, inci Name:, iodine trichloride, iron picolinate, isopropyl titanium triisostearate, isostearamidopropyl dimethylamine gluconate, isostearamidopropyl dimethylamine glycolate, isostearamidopropyl dimethylamine lactate, isostearamidopropyl morpholine lactate, lanthanum chloride, lauryl isoquinolinium saccharinate, lead acetate, lithium carbonate, lithium chloride, lithium fluoride, lithium gluconate, lithium magnesium silicate, lithium myristate, lithium oxidized polyethylene, lithium sulfide, luminescent zinc sulfide, lysine glutamate, magnesium acetate, magnesium aluminometasilicate, magnesium aluminum silicate, magnesium ascorbate, magnesium ascorbate/PCA, magnesium ascorbyl phosphate, magnesium ascorbylborate, magnesium benzoate, magnesium bromide, magnesium carbonate, magnesium carbonate hydroxide, magnesium chloride, magnesium citrate, magnesium fluoride, magnesium fluorosilicate, magnesium glucoheptonate, magnesium gluconate, magnesium glycerophosphate, magnesium hydrogen phosphate, magnesium lactate, magnesium laureth-11 carboxylate, magnesium lithospermate b, magnesium PCA, magnesium phosphate, magnesium propionate, magnesium salicylate, magnesium silicate, magnesium sulfate, magnesium sulfide, magnesium thioglycolate, magnesium trisilicate, manganese adenosine triphosphate, manganese chloride, manganese citrate, manganese gluconate, manganese glycerophosphate, manganese PCA, manganese sulfate, manganese Violet, mea o-phenylphenate, mea ppg-6 laureth-7 carboxylate, mea-benzoate, mea-biotinate, mea-cocoate, mea-dicetearyl phosphate, mea-laureth-6 carboxylate, mea-ppg-8-steareth-7 carboxylate, mea-salicylate, mea-thiolactate, mea-undecylenate, methyl aminolevulinate hcl, methyl hydroxycetyl glucaminium lactate, methylsilanol hydroxyproline aspartate, mineral salts, molybdenum aspartate, molybdenum chloride, monosodium citrate, niacinamide ascorbate, niacinamide hydroxybenzoate, niacinamide salicylate, niacinamide thioctate, nickel bis (Hydroxy diphenyl methyl pyrrolidino methyl) Pyridinediyl t-butylisocyano perchlorate, nickel chloride, nickel gluconate, nicotinyl tartrate, olivamidopropyl dimethylamine lactate, oxyquinoline benzoate, oxyquinoline sulfate, palmitoyl tripeptide-1 acetate, PCA Ethyl cocoyl arginate, pentapeptide-34 trifluoroacetate, pentapotassium triphosphate, pentasodium ethylenediamine tetramethylene phosphonate, pentasodium pentetate, pentasodium tetracarboxymethyl acetylhydroxyprolyl dipeptide-12, pentasodium tetracarboxymethyl dipeptide-51, pentasodium tetracarboxymethyl heptadecanoyl dipeptide-12, pentasodium triphosphate, phenyl mercuric acetate, phenyl mercuric benzoate, phenyl mercuric borate, phenyl mercuric bromide, phenyl mercuric chloride, phosphate buffered saline, phytin, piroctone olamine, poly[oxymethylene melamine acrylates/Acrylamide], potassium acesulfame, potassium acetate, potassium alum, potassium ascorbate, potassium ascorbyl tocopheryl phosphate, potassium ascorbylborate, potassium azeloyl diglycinate, potassium benzoate, potassium bicarbonate, potassium biphthalate, potassium bitartrate, potassium borate, potassium bromate, potassium bromide, potassium butylparaben, potassium C12-13 alkyl phosphate, potassium calcium silver phosphates extract, potassium carbomer, potassium carbonate, potassium caroate, potassium cellulose succinate, potassium chlorate, potassium chloride, potassium citrate, potassium cocoyl glutamate, potassium cocoyl glycinate, potassium cocoyl PCA, potassium cyanate, potassium ethylparaben, potassium fluoride, potassium fluorosilicate, potassium fructoborate, potassium glucoheptonate, potassium gluconate, potassium glycerophosphate, potassium glycol sulfate, potassium glycyrrhetinate, potassium glycyrrhizinate, potassium hydroxycitrate, potassium iodide, potassium jojobate, potassium lactate, potassium lanolate, potassium laureth-10 carboxylate, potassium laureth-3 carboxylate, potassium laureth-4 carboxylate, potassium laureth-5 carboxylate, potassium laureth-6 carboxylate, potassium lauroyl methyl beta-alanine, potassium lauroyl PCA, potassium lauryl phosphate, potassium metabisulfite, potassium methoxycinnamate, potassium methylparaben, potassium monofluorophosphate, potassium monopersulfate, potassium naphthyl methylhydroxydioxobenzothiazinolate methanone, potassium nitrate, potassium octatrienoate, potassium olivoyl PCA, potassium o-phenylphenate, potassium oxidized microcrystalline wax, potassium palmitoyl ascorbyl glucoside, potassium paraben, potassium PCA, potassium Peg-50 hydrogenated castor oil succinate, potassium perfluorohexyl ethylphosphate, potassium persulfate, potassium phenoxide, potassium phenylbenzimidazole sulfonate, potassium phosphate, potassium polyphosphate, potassium propionate, potassium propylparaben, potassium salicylate, potassium shellac, potassium silicate, potassium sodium tartrate, potassium sorbate, potassium succinate, potassium sulfate, potassium sulfide, potassium sulfite, potassium tartrate, potassium taurate, potassium taurine laurate, potassium tetrathionate, potassium thiocyanate, potassium thioglycolate, potassium trideceth-15 carboxylate, potassium trideceth-19 carboxylate, potassium trideceth-3 carboxylate, potassium trideceth-4 carboxylate, potassium trideceth-7 carboxylate, potassium troclosene, potassium undecylenoyl alginate, propylenediammonium dimaleate, pyridoxine hcl, pyridoxine hydroxycitrate, pyridoxine salicylate, saccharated lime, sea salt, selenium sulfide, silver benzoate, silver carboxymethylalaninate, silver chloride, silver citrate, silver lactate, silver methylmercaptopyrimidine, silver nitrate, silver salicylate, silver sulfate, sodium 3-hydroxybutyrate, sodium 5-nitroguaiacolate, sodium acetate, sodium allantoin PCA, sodium alum, sodium aluminate, sodium aluminum chlorohydroxy lactate, sodium aluminum lactate, sodium amps, sodium anisate, sodium arachidate, sodium ascorbate, sodium ascorbyl glucoside, sodium ascorbyl maltoside, sodium ascorbyl phosphate, sodium ascorbyl phosphate succinoyl hydrolyzed collagen, sodium behenoyl lactylate, sodium benzoate, sodium bicarbonate, sodium bis-(Laurylglucosides hydroxypropyl) Citrate, sodium bisulfate, sodium bisulfite, sodium borate, sodium borohydride, sodium bromate, sodium butoxyethoxy acetate, sodium butylparaben, sodium C11-15 alketh-7 carboxylate (Formerly sodium C11-15 pareth-7 carboxylate), sodium C12-13 alketh-12 carboxylate (Formerly sodium C12-13 pareth-12 carboxylate), sodium C12-13 alketh-5 carboxylate (Formerly sodium C12-13 pareth-5 carboxylate), sodium C12-13 alketh-8 carboxylate (Formerly sodium C12-13 pareth-8 carboxylate), sodium C12-14 sec-alketh-8 carboxylate (Formerly sodium C12-14 sec-pareth-8 carboxylate), sodium C12-15 alketh-12 carboxylate (Formerly sodium C12-15 pareth-12 carboxylate), sodium C12-15 alketh-6 carboxylate (Formerly sodium C12-15 pareth-6 carboxylate), sodium C12-15 alketh-7 carboxylate (Formerly sodium C12-15 pareth-7 carboxylate), sodium C12-15 alketh-8 carboxylate (Formerly sodium C12-15 pareth-8 carboxylate), sodium C14-15 alketh-8 carboxylate (Formerly sodium C14-15 pareth-8 carboxylate), sodium C9-11 alketh-6 carboxylate (Formerly sodium C9-11 pareth-6 carboxylate), sodium calcium boron phosphate, sodium calcium copper phosphate, sodium calcium silver phosphate, sodium calcium zinc phosphate, sodium caproyl lactylate, sodium caproyl/Lauroyl lactylate, sodium caproylethylformyl benzenesulfonate, sodium capryleth-2 carboxylate, sodium capryleth-9 carboxylate, sodium carbomer, sodium carbonate, sodium carbonate peroxide, sodium carboxydecyl Peg-8 dimethicone, sodium carboxyethyl tallow polypropylamine, sodium carboxymethyl C10-16 alkyl glucoside, sodium carboxymethyl cocopolypropylamine, sodium carboxymethyl euglena gracilis extract, sodium carboxymethyl inulin, sodium carboxymethyl oleyl polypropylamine, sodium carboxymethyl starch, sodium carboxymethyl tallow polypropylamine, sodium ceteareth-13 carboxylate, sodium ceteth-13 carboxylate, sodium chenodeoxycholate, sodium chlorate, sodium chloride, sodium chlorophyllin-zinc complex, sodium cholate, sodium cinnamate, sodium citrate, sodium citronellate, sodium coco sulfoacetate, sodium coco-glucoside tartrate, sodium cocoyl glycinate, sodium cocoyl hydrolyzed Royal jelly, sodium cocoyl lactylate, sodium cocoyl methyl beta-alanine, sodium cupheoyl lactylate, sodium cyanate, sodium cyclamate, sodium cyclodextrin butylsulfonate, sodium cyclodextrin sulfate, sodium cyclopentane carboxylate, sodium deceth-2 carboxylate, sodium dehydroacetate, sodium deoxycholate, sodium dilaureth-7 citrate, sodium dimyristoyl glyceryl ascorbate, sodium dipalmitoyl glycerophosphate, sodium distearoyl glyceryl ascorbate, sodium erythorbate, sodium ethylhexylglyceryl ascorbate, sodium ethylparaben, sodium ferrocyanide, sodium ferrous citrate, sodium ferulate, sodium fluoride, sodium fluorosilicate, sodium formate, sodium fructoborate, sodium fumarate, sodium gluceptate, sodium gluconate, sodium glucuronate, sodium glycerophosphate, sodium glycolate, sodium glyoxylate, sodium hexametaphosphate, sodium hexeth-4 carboxylate, sodium hinokitiol, sodium humate, sodium hydrolyzed casein, sodium hydrolyzed corn starch dodecenylsuccinate, sodium hydrosulfite, sodium hydroxymethane sulfonate, sodium hydroxypropyl oxidized starch succinate, sodium hydroxypyranonylmethyl sulfothioate, sodium iodate, sodium iodide, sodium isobutylparaben, sodium isoferulate, sodium isopropylparaben, sodium isosteareth-11 carboxylate, sodium isosteareth-6 carboxylate, sodium isostearoyl lactate, sodium isostearoyl lactylate, sodium lactate, sodium lactate methylsilanol, sodium laureth-11 carboxylate, sodium laureth-12 carboxylate, sodium laureth-13 carboxylate, sodium laureth-14 carboxylate, sodium laureth-16 carboxylate, sodium laureth-17 carboxylate, sodium laureth-3 carboxylate, sodium laureth-4 carboxylate, sodium laureth-5 carboxylate, sodium laureth-6 carboxylate, sodium laureth-7 tartrate, sodium laureth-8 carboxylate, sodium lauroyl aspartate, sodium lauroyl lactylate, sodium lauroyl/Myristoyl aspartate, sodium lauryl glucose carboxylate, sodium lauryl glycol carboxylate, sodium levulinate, sodium magnesium aluminum silicate, sodium magnesium silicate, sodium malate, sodium mannitylphytate, sodium mannuronate methylsilanol, sodium menthyl succinate, sodium metabisulfite, sodium metaphosphate, sodium metasilicate, sodium methoxy ppg-2 acetate, sodium methylesculetin acetate, sodium methylparaben, sodium molybdate, sodium monofluorophosphate, sodium myristoyl methyl beta-alanine, sodium naphthol sulfonate, sodium nitrate, sodium nitrite, sodium oleanolate, sodium oleoyl lactylate, sodium o-phenylphenate, sodium oxalate, sodium oxidized cellulose, sodium oxymethylene sulfoxylate, sodium palmitoyl ascorbyl glucoside, sodium pantetheine sulfonate, sodium pantothenate, sodium paraben, sodium PCA, sodium PCA methylsilanol, sodium p-chloro-m-cresol, sodium Peg-3 lauramide carboxylate, sodium Peg-4 cocamide sulfate, sodium Peg-4 lauramide carboxylate, sodium Peg-50 hydrogenated castor oil succinate, sodium Peg-6 cocamide carboxylate, sodium Peg-7 Olive oil carboxylate, sodium Peg-8 cocamide carboxylate, sodium Peg-8 palm glycerides carboxylate, sodium perborate, sodium perfluorohexyl ethylphosphonate, sodium persulfate, sodium pg-sulfonate, sodium phenoxide, sodium phenylbenzimidazole sulfonate, sodium phosphinate, sodium phytate, sodium picramate, sodium piperazinoethyl acetate ethylsulfonate, sodium polyaspartate, sodium polyphosphate, sodium potassium aluminum silicate, sodium propionate, sodium propoxy ppg-2 acetate, sodium propylparaben, sodium pyrithione, sodium pyruvate, sodium riboflavin phosphate, sodium saccharin, sodium salicylate, sodium sesquicarbonate, sodium silicate, sodium silicate extract, sodium silicoaluminate, sodium silver mercaptobenzimidazole sulfonate, sodium silver thioctoyl hyaluronate, sodium sorbate, sodium stannate, sodium stearoyl lactylate, sodium stearyl fumarate, sodium stearyl phthalamate, sodium succinate, sodium succinoyl gelatin, sodium sucrose octasulfate, sodium sulfate, sodium sulfide, sodium sulfite, sodium taurine laurate, sodium tetrahydrojasmonate, sodium thioctoyl hyaluronate, sodium thiocyanate, sodium thioglycolate, sodium thiosulfate, sodium trehalose octenylsuccinate, sodium trichloroacetate, sodium trideceth-12 carboxylate, sodium trideceth-15 carboxylate, sodium trideceth-19 carboxylate, sodium trideceth-3 carboxylate, sodium trideceth-4 carboxylate, sodium trideceth-6 carboxylate, sodium trideceth-7 carboxylate, sodium trideceth-8 carboxylate, sodium trimetaphosphate, sodium tungstate antimonate, sodium undeceth-5 carboxylate, sodium ursolate, sodium usnate, sodium zinc cetyl phosphate, sodium zinc histidine dithiooctanamide, sodium/Aluminum hydroxide/Oxalate/Sulfate, sodium/ Aluminum/Iron hydroxide/Oxalate/Sulfate, sodium/Aluminum/Iron/Sulfate/Citrate/Hydroxide, sodium/Aluminum/Iron/Sulfate/Oxalate/Hydroxide, sodium/Aluminum/Iron/Sulfate/Tartarate/Hydroxide, sodium/Tea C12-13 alketh-3 sulfate (Formerly sodium/Tea C12-13 pareth-3 sulfate), sodium/Tea-undecylenoyl alginate, sodium/Tea-undecylenoyl carrageenan, sorbitan hexa-guanidinohexanoate trifluoroacetate, stannous chloride, stannous fluoride, stannous pyrophosphate, strontium acetate, strontium chloride, strontium chloride hexahydrate, strontium citrate, strontium sulfide, strontium thioglycolate, t-butylhydroxylamine acetate, tea-carbomer, tea-edta, tea-glyceryl dimaleate, tea-lauroyl lactylate, tea-lauroyl/Myristoyl aspartate, tea-Peg-50 hydrogenated castor oil succinate, tea-phenylbenzimidazole sulfonate, terbium nitrate, tetracarboxymethyl dipeptide-12, tetrapotassium etidronate, tetrapotassium pyrophosphate, tetrasodium disuccinoyl cystine, tetrasodium edta, tetrasodium etidronate, tetrasodium glutamate diacetate, tetrasodium hydroxy iminodisuccinate, tetrasodium iminodisuccinate, tetrasodium pyrophosphate, tetrasodium tetracarboxymethyl naringeninchalcone, thiamine bis-laurylsulfate, thimerosal, thurfylnicotinate hcl, titanium bis(Hydrogen phosphate), titanium citrate, titanium ethoxide, titanium isostearates, titanium salicylate, tocopheryl tranexamate hcl, tricalcium phosphate, triferric aqua hydroxy oxo bis-trimesate, trimagnesium phosphate, tripotassium edta, tripotassium glycyrrhizate, tripotassium phosphate, tripotassium ursolyl phosphate, tris(Tetramethylhydroxypiperidinol) Citrate, trisodium chlorin E6, trisodium copper chlorin E6, trisodium dicarboxymethyl alaninate, trisodium fructose diphosphate, trisodium glycyrrhizate, trisodium inositol triphosphate, trisodium phosphate, trisodium sulfosuccinate, zinc acetate, zinc adenosine triphosphate, zinc adenosine triphosphate hydroxide, zinc ascorbate, zinc ascorbate hydroxide, zinc azelate hydroxide, zinc borate, zinc carbonate, zinc carbonate hydroxide, zinc carboxydecyl trisiloxane, zinc chloride, zinc citrate, zinc cysteinate, zinc dibutyldithiocarbamate, zinc dimethicone Peg-25 phthalate, zinc dimethicone Peg-8 succinate, zinc docosahexaenoate hydroxide, zinc eicosapentaenoate hydroxide, zinc ethylhexanoate, zinc formaldehyde sulfoxylate, zinc glucoheptonate, zinc gluconate, zinc glycyrrhetinate, zinc hexametaphosphate, zinc hydrolyzed hyaluronate, zinc indoleacetate hydroxide, zinc isomerized linoleate hydroxide, zinc lactate, zinc linoleate hydroxide, zinc linolenate hydroxide, zinc nitrate, zinc PCA, zinc phosphate, zinc picolinate, zinc pyrithione, zinc retinoate hydroxide, zinc salicylate, zinc silicate, zinc sulfate, zinc sulfide, zinc thiosalicylate, zirconium bis(Hydrogen phosphate), zirconium chlorohydrate, zirconium oxide acetate trimesicate, zirconium silicate, zirconyl chloride, or a combination thereof.

According to some aspects, the cosmetic composition may include each of the one or more salts individually at a concentration between about 0.01 and 20% w/v, optionally between about 0.01 and 15% w/v, and optionally between about 0.1 and 10% w/v, including any value therebetween. According to some aspects, the cosmetic composition may include a total salt concentration of between about 0.01 and 20% w/v, optionally between about 0.01 and 15% w/v, and optionally between about 0.1 and 10% w/v, including any value therebetween.

The cosmetic composition of the present disclosure includes one or more colorants. As used herein, the term "colorant" refers to a component configured to provide color, opacity, and/or iridescence to a composition as described herein. Example colorants include, but are not limited to, iron oxide, titanium dioxide, titanium coated mica minerals, mica minerals, talc, bismuth oxychloride, pigmented pearls, FD&C and D&C colors, mango violet, ultra marine colors, chrome oxide colors, carmine, ferric ammonium ferrocyanide, and combinations thereof.

According to some aspects, the one or more colorants may include aluminum powder (CI 77000), annatto (CI 75120), beta-carotene (CI 40800), beta-carotene (CI 75130), bismuth oxychloride (CI 77163), black 2 (CI 77266), black 3 (CI 77266), blue 1 (CI 42090), blue 1 lake (CI 42090), blue 4 (CI 42090), bronze powder (CI 77400), brown 1 (CI 20170), caramel, Carmine (CI 75470), chlorophyllin-copper complex (CI 75810), chromium hydroxide green (CI 77289), chromium oxide greens (CI 77288), copper powder (CI 77400), ext. Yellow 7 (CI 10316), ext. Yellow 7 lake (CI 10316), ferric ammonium ferrocyanide (CI 77510), ferric ferrocyanide (CI 77510), green 3 (CI 42053), green 3 lake (CI 42053), green 5 (CI 61570), green 6 (CI 61565), green 8 (CI 59040), guanine (CI 75170), iron oxides (CI 77491), iron oxides (CI 77492), iron oxides (CI 77499), manganese Violet (CI 77742), orange 10 (CI 45425), orange 10 lake (CI 45425), orange 11 (CI 45425), orange 4 (CI 15510), orange 4 lake (CI 15510), orange 5 (CI 45370), orange 5 lake (CI 45370), red 17 (CI 26100), red 21 (CI 45380 ), red 21 lake (CI 45380 ), red 22 (CI 45380), red 22 lake (CI 45380), red 27 (CI 45410), red 27 lake (CI 45410), red 28 (CI 45410), red 28 lake (CI 45410), red 30 (CI 73360), red 30 lake (CI 73360), red 31 (CI 15800), red 31 lake (CI 15800), red 33 (CI 17200), red 33 lake (CI 17200), red 34 (CI 15880), red 34 lake (CI 15880), red 36 (CI 12085), red 36 lake (CI 12085), red 4 (CI 14700), red 4 lake (CI 14700), red 40 (CI 16035), red 40 lake (CI 16035), red 6 (CI 15850), red 6 lake (CI 15850), red 7 (CI 15850), red 7 lake (CI 15850), silver (CI 77820), titanium dioxide (CI 77891), ultramarines (CI 77007), Violet 2 (CI 60725), yellow 10 (CI 47005), yellow 10 lake (CI 47005), yellow 11 (CI 47000), yellow 5 (CI 19140), yellow 5 lake (CI 19140), yellow 6 (CI 15985), yellow 6 lake (CI 15985), yellow 7 (CI 45350), yellow 7 lake (CI 45350), yellow 8 (CI 45350), zinc oxide (CI 77947), pyrophyllite, guaiazulene, one or more mica minerals, black 3 (CI 77267), or a combination thereof.

According to some aspects, the one or more colorants may include lactoflavin, riboflavin, benzimidazole Diamond amidoethyl urea carbamoyl propyl polymethylsilsesquioxane, aluminum stearate, anthocyanins, beetroot red, calcium stearate, capsanthin/Capsorubin, magnesium carbonate (CI 77713), calcium carbonate (CI 77220) , calcium sulfate (CI 77231) , barium sulfate (CI 77120) , magnesium stearate, zinc stearate, bismuth citrate, barium sulfate, charcol powder, CI 77480 (Gold), acid red 195, (CI 77346) Cobalt aluminum oxide, curcumin (CI 75300), lycopene (CI 75125), crocetin (CI 75100), CI 74260, CI 45430, CI 10316, CI 11680, CI 11710, CI 11920, CI 12010, CI 12490, CI 13015, CI 14270, CI 14720, CI 14815, CI 15525, CI 15580, CI 15630, CI 15865, CI 15980, CI 16185, CI 16230, CI 16255, CI 16290, CI 18050, CI 18965, CI 21230 /Solvent yellow 29, CI 26100 /Solvent red 23, CI 27755, CI 28440, CI 40820, CI 40825, CI 40850, CI 42045 /Acid blue 1, CI 42051 /Acid blue 3, CI 42053/ Fast green fcf, CI 42090/ Acid blue 9; Acid blue 9 ammonium salt; Acid blue 9 aluminum lake, CI 42510, CI 42735, CI 44045, CI 44090, CI 45380 / Acid red 87, CI 45396, CI 45405, CI 45430, CI 47000 / Solvent yellow 33, CI 50420, CI 58000 / Pigment red 83, CI 69800, CI 69825, CI 71105, CI 73000, CI 73015, CI 73385, CI 74160, CI 74260, CI 75100, CI 75125, CI 75135, CI 75300, CI 77002, CI 77004, CI 77015, CI 77266, CI 77267, CI 77268:1, CI 77346, CI 77480, or a combination thereof.

According to some aspects, each of the one or more of the colorants may individually be provided as particles with an average particle size between about 0.01 and 10,000 µm, optionally between about 1 and 5,000 µm.

According to some aspects, the cosmetic composition may include each of the one or more colorants individually at a concentration between about 0.000001 and 75% w/v, optionally between about 0.000001 and 50% w/v, including any value therebetween. According to some aspects, the cosmetic composition may include a total colorant concentration of between about 0.000001 and 75% w/v, optionally between about 0.000001 and 50% w/v, including any value therebetween.

According to some aspects, the cosmetic composition may include one or more bulking agents. As used herein, a "bulking agent" refers to a component that increases the volume of a cosmetic composition. It should be understood that one or more of the bulking agents described herein may also function as a colorant.

According to some aspects, the one or more bulking agents may include a silicate (e.g., aluminum calcium sodium silicate, calcium silicate, sodium magnesium silicate, sodium potassium aluminum silicate), a borosilicate (e.g., calcium aluminum borosilicate, calcium sodium borosilicate, calcium titanium borosilicate), glass, glass beads, gold, hydrated silica, a fluorosilicate (e.g., sodium magnesium fluorosilicate, magnesium fluorosilicate, magnesium potassium fluorosilicate, magnesium sodium fluorosilicate), one or more mica minerals, silica, synthetic fluorphlogopite, bismuth oxychloride, or a combination thereof.

According to some aspects, each of the one or more of the bulking agents may individually be provided as particles with an average particle size between about 0.01 and 10,000 µm, optionally between about 1 and 5,000 µm.

According to some aspects, the cosmetic composition may include each of the one or more bulking agent individually at a concentration between about 0.000001 and 85% w/v, optionally between about 0.000001 and 65% w/v, including any value therebetween. According to some aspects, the cosmetic composition may include a total colorant concentration of between about 0.000001 and 85% w/v, optionally between about 0.000001 and 65% w/v, including any value therebetween.

The cosmetic composition of the present disclosure includes one or more dispersants. As used herein, the term "dispersant" refers to a component configured to facilitate a homogenous dispersion of at least one other component of the cosmetic composition within a solvent. According to some aspects, the one or more dispersants may include a glycol. As used herein, the term "glycol" refers to a diol having from 2 to 8 carbon atoms. Non-limiting glycols include propylene glycol, ethylene glycol, 1,3-butylene glycol, dipropylene glycol, 1,2-propanediol, 1,3-propanediol, 1,1-propanediol, 2,2-propanediol, and combinations thereof.

According to some aspects, the cosmetic composition may include each dispersant individually at a concentration of between about 0.01 and 50% w/v, optionally between about 0.01 and 25% w/v, optionally between about 0.1 and 20% w/v, optionally between about 0.1 and 10% w/v, and optionally between about 0.1 and 5% w/v, including any value therebetween. According to some aspects, the cosmetic composition may include a total dispersant concentration of between about 0.01 and 50% w/v, optionally between about 0.01 and 25% w/v, optionally between about 0.1 and 20% w/v, optionally between about 0.1 and 10% w/v, and optionally between about 0.1 and 5% w/v, including any value therebetween.

The cosmetic composition of the present disclosure may further include one or more preservatives. As used herein, the term "preservative" refers to a component configured to extend the shelf life of a cosmetic composition in relation to the shelf life of the cosmetic composition without the preservative. According to some aspects, the one or more preservatives may include a glycol (such as pentylene glycol, caprylyl glycol, decylene glycol, and combinations thereof), natural preservatives, phenoxyethanol, and combinations thereof. It should be understood that the one or more preservatives of the present disclosure may perform at least two functions as described herein, such as the function of both a preservative and a dispersant.

According to some aspects, the cosmetic composition may include each preservative individually at a concentration of between about 0.01 and 50% w/v, optionally between about 0.01 and 25% w/v, optionally between about 0.1 and 20% w/v, optionally between about 0.1 and 10% w/v, and optionally between about 0.1 and 5% w/v, including any value therebetween. According to some aspects, the cosmetic composition may include a total preservative concentration of between about 0.01 and 50% w/v, optionally between about 0.01 and 30% w/v, optionally between about 0.01 and 25% w/v, optionally between about 0.1 and 20% w/v, optionally between about 0.1 and 10% w/v, and optionally between about 0.1 and 5% w/v, including any value therebetween.

The cosmetic composition of the present disclosure includes a pH adjuster. As used herein, the term "pH adjuster" refers to a component or combination of components sufficient to adjust a cosmetic composition's pH. According to some aspects, the pH adjuster is sufficient to raise or lower the cosmetic composition's initial pH to a cosmetically acceptable range (e.g., to provide acceptable stability and/or structure). Additionally or alternatively, the pH adjuster may be sufficient to maintain the cosmetic composition's pH in an acceptable range over a certain period of shelf life. According to some aspects, the cosmetically acceptable pH range may be between about 3.5 and 9.5, optionally between about 4 and 9, and optionally between about 4.5 and 8.5.

According to some aspects, the one or more pH adjusters may include 2-aminobutanol, acetic acid, acetyl mandelic acid, adipic acid, aluminum hydroxide oxide, aluminum triformate, aminoethyl propanediol, aminomethyl propanediol, aminomethyl propanol, aminopropanediol, aminopropanol, ammonia, ammonium bicarbonate, ammonium carbamate, ammonium carbonate, ammonium glycolate, ammonium hydroxide, ammonium phosphate, anadara kagoshimensis shell powder, arginine, ascorbic acid, asterionellopsis glacialis extract, azelaic acid, babassu acid, bakuhan, benzilic acid, benzoic acid, bis-hydroxyethyl tromethamine, brown rice vinegar, butyl diethanolamine, butylethanolamine, calcium citrate, calcium dihydrogen phosphate, calcium glycinate, calcium hydroxide, calcium oxide, camphor sulfonic acid, campomanesia lineatifolia fruit extract, Candida (Lactis-condensi/ Robusta/Utilis)/ Debaryomyces/Lactobacillus/Leuconostoc/Pichia/Saccharomyces/Streptococcus (Cremoris/Faecalis/Lactis/Thermophilus)/ Torulaspora delbrueckii/Zygosaccharomyces ferment extract, chaetoceros constrictus/Debilis/Decipiens/Laciniosus/Socialis extract, chloroacetic acid, citrate buffer, citric acid, Clay minerals, coconut flower sugar, cocos nucifera (Coconut) Flower nectar extract, copper glycinate, cysteine hcl, dactyliosolen fragilissimus extract, detonula pumila extract, dibutyl ethanolamine, diethanolamine, diethanolamine bisulfate, dihydroxyacetic acid, diisopropanolamine, diisopropylamine, dimethyl isopropanolamine, dimethyl mea, dimethylglucamine, dioleoyl edetolmonium methosulfate, dioleyl phosphate, dipotassium phosphate, dipropylenetriamine, disodium fumarate, disodium phosphate, disodium pyrophosphate, disodium tartrate, ditylum brightwellii extract, ethanolamine, ethanolamine hcl, Ethyl ethanolamine, formic acid, fumaric acid, galacturonic acid, garcinia cambogia peel extract, geranic acid, glucoheptonic acid, glucosamine hcl, glucuronic acid, glutaric acid, glycolic acid, glyoxylic acid, guanidine carbonate, hydrobromic acid, hydrochloric acid, imidazole, isopropanolamine, isopropylamine, itaconic acid, ketoglutaric acid, lactic acid, lactobacillus/Amaranthus caudatus seed flour ferment filtrate, lactobacillus/Laminaria japonica/Molasses ferment filtrate, lactobacillus/Opuntia ficus-indica stem/Molasses ferment filtrate, lactobacillus/Pyrus communis (Pear) Fruit/Molasses ferment filtrate, lactobionic acid, leptocylindrus danicus extract, lithium carbonate, lithium hydroxide, magnesium carbonate, magnesium carbonate hydroxide, magnesium glycinate, magnesium hydroxide, magnesium oxide, maleic acid, malic acid, malonic acid, maltobionic acid, metaphosphoric acid, methoxy Peg-100/Polyepsilon caprolactone ethylhexanoate, methoxy Peg-100/Polyepsilon caprolactone palmitate, methoxy Peg-114/Polyepsilon caprolactone, methylethanolamine, methylglucamine, mixed isopropanolamines, monosodium citrate, morpholine, morpholinopropane sulfonic acid, nickel chloride, oxalic acid, paecilomyces japonicalGrape/Cucumber juice extract ferment filtrate, p-anisic acid, pentapotassium triphosphate, pentasodium triphosphate, phenolsulfonphthalein, phosphoric acid, plantago ovata husk powder, potassium bicarbonate, potassium biphthalate, potassium borate, potassium carbonate, potassium citrate, potassium hydroxide, potassium magnesium aspartate, potassium nitrate, potassium oxide, potassium phosphate, potassium tartrate, potassium vanadyl tungstate antimonate, propionibacterium acidipropionici/Soybean extract ferment filtrate, propionic acid, quinic acid, rhizosolenia setigera extract, ribonic acid, Rosa damascena petal/Molasses vinegar, sarcothalia crispata, s-dna aptamer-1, s-dna aptamer-2, sebacic acid, sesquiethoxytriethanolamine, sodium 3-mercaptopropane sulfonate, sodium aluminate, sodium anisate, sodium arachidate, sodium bicarbonate, sodium bisulfate, sodium borate, sodium calcium boron phosphate, sodium calcium copper phosphate, sodium calcium zinc phosphate, sodium carbonate, sodium cinnamate, sodium citrate, sodium cyanate, sodium fumarate, sodium glycolate, sodium hydroxide, sodium oxide, sodium sesquicarbonate, sodium silicate, sodium succinate, sodium trimetaphosphate, sodium tungstate antimonate, sorbityl dimonium anisate, strontium hydroxide, succinic acid, sulfonated styrene/Dvb crosspolymer, sulfuric acid, tartaric acid, tea-diricinoleate/Ipdi copolymer, tetrapotassium pyrophosphate, tetrasodium pyrophosphate, thalassiosira aestivalis/Gravida/Nitzschioides/Nordenskioeldii/Rotula extract, triethanolamine, trifluoroacetic acid, triisopropanolamine, tripeptide-51, tripotassium phosphate, trisodium phosphate, trisodium sulfosuccinate, tromethamine, vinegar, zinc carbonate hydroxide, zinc glycinate, zinc magnesium aspartate, alstonia scholaris bark extract, aluminum glycinate, aluminum lactate, ammonium acetate, ammonium hexafluorophosphate, ammonium lactate, ammonium molybdate, ammonium vanadate, aspergillus/Defatted soybean seed ferment extract, bis-methoxy Peg-114 polycaprolactone diol/Ipdi copolymer, boric acid, calcium carbonate, calcium phosphate, cyclohexylamine, defined cell culture media 17, diammonium citrate, diammonium phosphate, diethyl ethanolamine, diethylamine, disodium itaconate, dmem, dulbecco's phosphate buffered saline, ectoin, glycine, glycine hcl, hydroxyectoin, hydroxyethylpiperazine ethane sulfonic acid, lauryl p-cresol ketoxime, lithium fluoride, magnesium acetate, magnesium lactate, mea-borate, mipa-borate, mudstone powder, phosphonobutanetricarboxylic acid, polyglyceryl-25/Caprolactone, potassium acetate, potassium bitartrate, potassium lactate, ppg-70 bis-(2-aminopropyl) Ether, sh-decapeptide-7, sodium acetate, sodium aluminum lactate, sodium ascorbyl maltoside, sodium esylate, sodium humate, sodium lactate, sodium pentaerythrityl hydroxypropyl iminodiacetate dendrimer, sodium phosphate, tris buffered saline, urea, zinc hexametaphosphate, zinc hydroxide, or a combination thereof.

According to some aspects, the cosmetic composition may include each pH adjuster individually at a concentration of between about 0.000001 to 5% v/v, optionally between about 0.000001 and 4% v/v, optionally between about 0.000001 and 3% v/v, and optionally between about 0.000001 and 2% v/v, including any value therebetween. According to some aspects, the cosmetic composition may include a total pH adjuster concentration of between about 0.000001 to 5% v/v, optionally between about 0.000001 and 4% v/v, optionally between about 0.000001 and 3% v/v, and optionally between about 0.000001 and 2% v/v, including any value therebetween.

The cosmetic composition further includes a solvent having one or more solvent components. According to some aspects, the one or more solvent components includes water.

According to some aspects, the cosmetic composition may include each solvent component individually at a concentration of between about 0.01 and 99.99% v/v, and optionally between about 0.1 and 99% v/v, including any value therebetween. According to some aspects, the cosmetic composition may include a total solvent concentration of between about 0.01 and 99.99% v/v, and optionally between about 0.1 and 99% v/v, including any value therebetween. According to some aspects, the cosmetic composition may include a total solvent concentration of q.s. to 100% v/v.

The cosmetic composition according to the present disclosure is not particularly limited and may include, for example, a skin lotion, skin milk, skin cream, gel, foundation, foundation primer base, blush, lip stick, eye shadow, eye liner, nail enamel, concealer, mascara, body make-up product, sunscreen, or a combination thereof. The cosmetic composition may have a structure that is suitable for any one of the examples as described herein, such as a solid (e.g., a stick), a liquid, a gel, a powder, an emulsion, or a combination thereof.

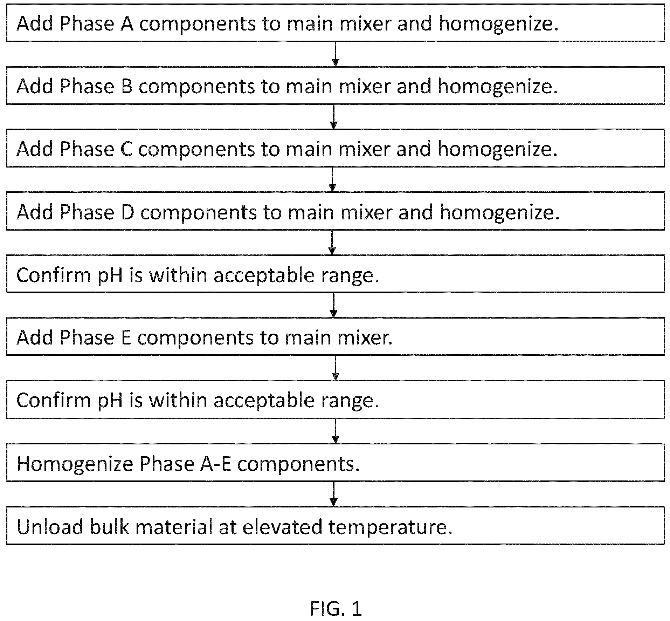

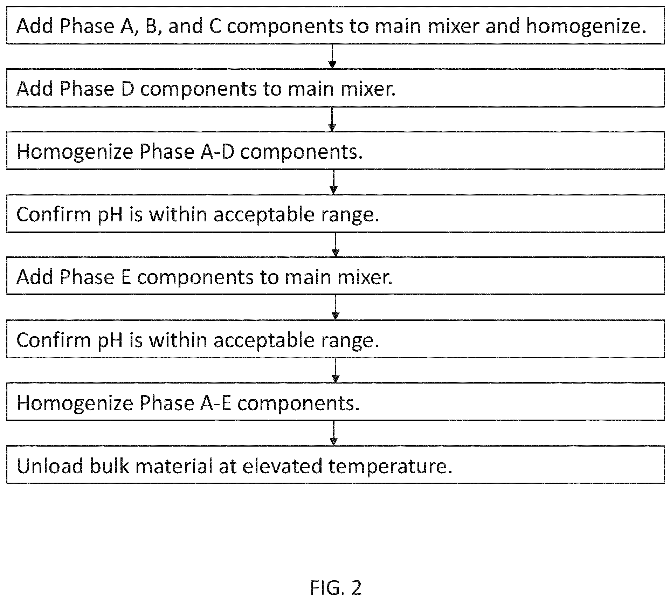

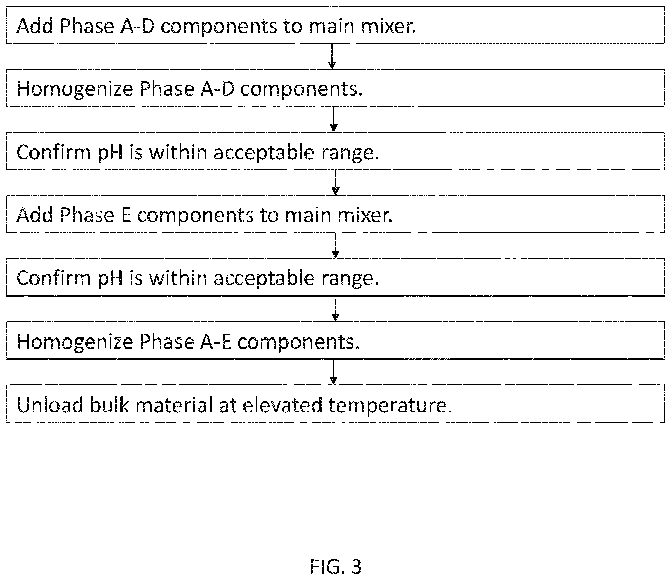

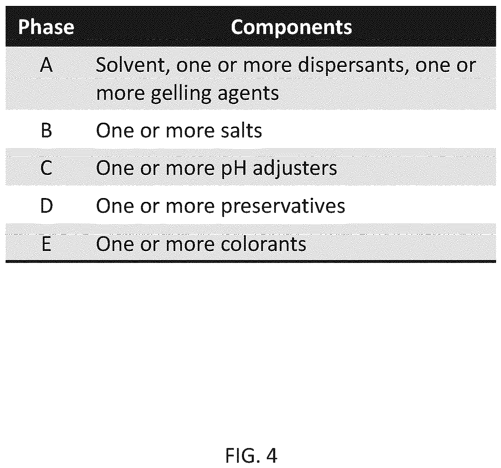

The present disclosure is also directed to methods of making the cosmetic composition as described herein. FIG. 1 shows the steps of one example method as described herein. FIG. 2 shows the steps of another example method as described herein. FIG. 3 shows the steps of another example method as described herein. FIG. 4 shows example components of the phases shown in FIGs. 1-3.

It should be understood that as used herein, the term "step" is not particularly limiting. For example, each step as described herein may be a discrete step such that steps are performed sequentially upon completion of a preceding step. Alternatively, at least a portion of the method may be continuous. According to some aspects, the order of the steps as described herein may provide acceptable stability and/or structure to the cosmetic composition provided thereby.

According to some aspects, the method may include combining the solvent, the one or more dispersants, and the one or more gelling agents to provide a first combination. It should be understood the solvent, the one or more dispersants, and the one or more gelling agents may be combined sequentially or simultaneously. Additionally or alternatively, the solvent, the one or more dispersants, and/or the one or more gelling agents may be combined in batches.

According to some aspects, the one or more solvent components, the one or more dispersants, and the one or more gelling agents may be combined in an apparatus configured to provide a homogenous mixture. For example, the one or more solvent components, the one or more dispersants, and the one or more gelling agents may be combined in a mixer configured to homogeneously mix, alternatively referred to as "homogenize," the first combination. Alternatively, one or more of the one or more solvent components, the one or more dispersants, and the one or more gelling agents may be combined in a first container and transferred to a mixer.

According to some aspects, the first combination may be heated to an elevated temperature. The elevated temperature may be between about 60 and 90°C, optionally between about 65 and 85°C, optionally between about 70 and 80°C, optionally between about 71 and 79°C, optionally between about 72 and 78°C, optionally between about 73 and 77°C, optionally between about 74 and 76°C, and optionally about 75°C. According to some aspects, each component of the first combination may be individually heated to the elevated temperature prior to combination. Additionally or alternatively, two of more components of the first combination may be combined and subsequently heated to the elevated temperature, and/or two of more components of the first combination may be simultaneously combined and heated to the elevated temperature.

According to some aspects, the first combination may be homogenized for a period of time sufficient to provide a first homogenous mixture. In one example, the period of time may be between about 1 and 20 minutes, optionally between about 5 and 15 minutes, and optionally about 10 minutes. In another example, the period of time may be between about 10 and 30 minutes, optionally between about 15 and 25 minutes, and optionally about 20 minutes.

The method may include combining the first homogenous mixture with the one or more salts as described herein to provide a second combination.

According to some aspects the first homogenous mixture and the one or more salts may be combined sequentially or simultaneously. Additionally or alternatively, the first homogenous mixture and the one or more salts may be combined in batches.

According to some aspects, the first homogenous mixture and the one or more salts may be combined in the apparatus used to provide the first homogenous mixture, such as the mixer. In this example, the one or more salts may be combined prior to combination with the first homogenous mixture in the mixer.

According to some aspects, the second combination may be heated to an elevated temperature. The elevated temperature may be between about 60 and 90°C, optionally between about 65 and 85°C, optionally between about 70 and 80°C, optionally between about 71 and 79°C, optionally between about 72 and 78°C, optionally between about 73 and 77°C, optionally between about 74 and 76°C, and optionally about 75°C. According to some aspects, each component of the second combination may be individually heated to the elevated temperature prior to combination. Additionally or alternatively, two of more components of the second combination may be combined and subsequently heated to the elevated temperature, and/or two of more components of the second combination may be simultaneously combined and heated to the elevated temperature.

According to some aspects, the second combination may be homogenized for a period of time sufficient to provide a second homogenous mixture. In one example, the period of time may be between about 1 and 20 minutes, optionally between about 5 and 15 minutes, and optionally about 10 minutes.

The method may include combining the second homogenous mixture with one or more pH adjusters as described herein to provide a third combination.

According to some aspects the second homogenous mixture and the one or more pH adjusters may be combined sequentially or simultaneously. Additionally or alternatively, the solvent, the second homogenous mixture and the one or more pH adjusters may be combined in batches.

According to some aspects, the second homogenous mixture and the one or more pH adjusters may be combined in the apparatus used to provide the first and/or second homogenous mixture, such as the mixer. In this example, the one or more pH adjusters may be combined prior to combination with the second homogenous mixture in the mixer.

According to some aspects, the third combination may be heated to an elevated temperature. The elevated temperature may be between about 60 and 90°C, optionally between about 65 and 85°C, optionally between about 70 and 80°C, optionally between about 71 and 79°C, optionally between about 72 and 78°C, optionally between about 73 and 77°C, optionally between about 74 and 76°C, and optionally about 75°C. According to some aspects, each component of the third combination may be individually heated to the elevated temperature prior to combination. Additionally or alternatively, two of more components of the third combination may be combined and subsequently heated to the elevated temperature, and/or two of more components of the third combination may be simultaneously combined and heated to the elevated temperature.

According to some aspects, the third combination may be homogenized for a period of time sufficient to provide a third homogenous mixture. In one example, the period of time may be between about 1 and 20 minutes, optionally between about 5 and 15 minutes, and optionally about 10 minutes.

The method may include combining the third homogenous mixture with one or more preservatives as described herein to provide a fourth combination.

According to some aspects the third homogenous mixture and the one or more preservatives may be combined sequentially or simultaneously. Additionally or alternatively, the components may be combined in batches.

According to some aspects, the third homogenous mixture and the one or more preservatives may be combined in the apparatus used to provide the first, second, and/or third homogenous mixture, such as the mixer. In this example, the one or more preservatives may be combined prior to combination with the third homogenous mixture in the mixer.

According to some aspects, the fourth combination may be heated to an elevated temperature. The elevated temperature may be between about 60 and 90°C, optionally between about 65 and 85°C, optionally between about 70 and 80°C, optionally between about 71 and 79°C, optionally between about 72 and 78°C, optionally between about 73 and 77°C, optionally between about 74 and 76°C, and optionally about 75°C. According to some aspects, each component of the fourth combination may be individually heated to the elevated temperature prior to combination. Additionally or alternatively, two of more components of the fourth combination may be combined and subsequently heated to the elevated temperature, and/or two of more components of the fourth combination may be simultaneously combined and heated to the elevated temperature.

According to some aspects, the fourth combination may be homogenized for a period of time sufficient to provide a fourth homogenous mixture. In one example, the period of time may be between about 1 and 20 minutes, optionally between about 5 and 15 minutes, and optionally about 10 minutes.

The method of the present disclosure may include a pH monitoring step and/or a pH adjustment step. As used herein, a "pH monitoring step" refers to a step of determining the pH of any of the combinations, homogeneous mixtures, and/or components thereof as described herein as known in the art. The method may include one, two, three, four, or more pH monitoring steps.

According to some aspects, the method may further include a pH adjustment step if a pH as determined in the pH monitoring step is outside of a specified pH range. As used herein, the term "pH adjustment step" refers to a step for adjusting the pH of a combination, homogeneous mixture, and/or component thereof by adding one or more pH adjusters as described herein. It should be understood that if the pH of the combination, homogeneous mixture, and/or component thereof as determined by the pH monitoring step is outside of the specified range, one or more pH adjusters may added to the combination, homogeneous mixture, and/or component thereof until the pH is within the specified pH range. According to some aspects, at least one of the one or more pH adjusters used in the pH adjustment step may be the same as at least one of the one or more of the pH adjusters contained in the combination, homogeneous mixture, and/or component thereof. Additionally or alternatively, at least one of the one or more pH adjusters used in the pH adjustment step may be different from the pH adjusters contained in the combination, homogeneous mixture, and/or component thereof.

According to some aspects, the method includes at least a first pH monitoring step to determine the pH of the fourth homogenous mixture as described herein. In this example, the method may further include at least a first pH adjustment step if the pH as determined in the first pH monitoring step is outside of a first specified pH range. According to some aspects, the first specified pH range may be between about 7.5 and 10.5, optionally between about 8 and 10, optionally between about 8.1 and 9.9, optionally between about 8.2 and 9.8, optionally between about 8.3 and 9.7, optionally between about 8.4 and 9.6, and optionally between about 8.5 and 9.5.

The method may include combining the fourth homogenous mixture having a pH within the first specified pH range with the one or more colorants as described herein to provide a fifth combination.

According to some aspects, the fourth homogenous mixture and the one or more colorants may be combined sequentially or simultaneously. Additionally or alternatively, the fourth homogenous mixture and the one or more colorants may be combined in batches.

According to some aspects, the fourth homogenous mixture and the one or more colorants may be combined in the apparatus used to provide the first, second, third, and/or fourth homogenous mixture, such as the mixer. In this example, the one or more colorants may be combined prior to combination with the fourth homogenous mixture in the mixer.

According to some aspects, the fifth combination may be heated to an elevated temperature. The elevated temperature may be between about 60 and 90°C, optionally between about 65 and 85°C, optionally between about 70 and 80°C, optionally between about 71 and 79°C, optionally between about 72 and 78°C, optionally between about 73 and 77°C, optionally between about 74 and 76°C, and optionally about 75°C. According to some aspects, each component of the fifth combination may be individually heated to the elevated temperature prior to combination. Additionally or alternatively, two of more components of the fifth combination may be combined and subsequently heated to the elevated temperature, and/or two of more components of the fifth combination may be simultaneously combined and heated to the elevated temperature.

According to some aspects, the method includes at least a second pH monitoring step to determine the pH of the fifth combination as described herein. In this example, the method may further include at least a second pH adjustment step if the pH as determined in the second pH monitoring step is outside of a second specific pH range. According to some aspects, the second specified pH range may be between about 5 and 8, optionally between about 5.5 and 7.5, optionally between about 5.6 and 7.4, optionally between about 5.7 and 7.3, optionally between about 5.8 and 7.2, optionally between about 5.9 and 7.1, and optionally between about 6 and 7.

According to some aspects, the fifth combination having a pH within the second specific pH range may be homogenized for a period of time sufficient to provide a final homogenous mixture. In one example, the period of time may be between about 1 minute and 1 hour, optionally between about 15 and 45 minutes, and optionally about 30 minutes.

According to some aspects, at least a portion of any of the one or more of the steps described herein may be performed in a reduced pressure environment. As used herein, the term "reduced pressure environment" refers to an environment having a pressure that is below the average atmospheric pressure, for example, below 1013 mbar. According to some aspects, the reduced pressure may be between about 200 and 800 mbar, optionally between about 300 and 700 mbar, and optionally between about 500 and 600 mbar. In some examples wherein one or more steps are performed in a mixer as described herein, the mixer may be provided with a vacuum sufficient to provide a reduced pressure environment as described herein.

The method may further include removing the final homogenous mixture from the mixer to provide the cosmetic composition as described herein, also referred to herein as "unloading" the final homogenous mixture. According to some aspects, the final homogenous mixture may be unloaded from the mixer at an elevated temperature. The elevated temperature may be between about 50 and 100°C, optionally between about 50 and 70°C, and optionally between about 58 and 64°C.

The method may further include providing the cosmetic composition in a container. It should be understood that the container is not particularly limited and may be any container suitable for containing a cosmetic composition as described herein. According to some aspects, the container may include a material that is inert with respect to the cosmetic composition, such as a plastic. Example plastics useful according to the present disclosure include low density polyethylene (LDPE), high density polyethylene (HDPE), polypropylene (PP), and combinations thereof. Additionally or alternatively, the material may include silicon, glass, aluminum, or a combination thereof.

While the aspects described herein have been described in conjunction with the example aspects outlined above, various alternatives, modifications, variations, improvements, and/or substantial equivalents, whether known or that are or may be presently unforeseen, may become apparent to those having at least ordinary skill in the art. Accordingly, the example aspects, as set forth above, are intended to be illustrative, not limiting. Various changes may be made without departing from the spirit and scope of the disclosure. Therefore, the disclosure is intended to embrace all known or later-developed alternatives, modifications, variations, improvements, and/or substantial equivalents.

Thus, the claims are not intended to be limited to the aspects shown herein, but are to be accorded the full scope consistent with the language of the claims, wherein reference to an element in the singular is not intended to mean "one and only one" unless specifically so stated, but rather "one or more." All structural and functional equivalents to the elements of the various aspects described throughout this disclosure that are known or later come to be known to those of ordinary skill in the art are expressly incorporated herein by reference and are intended to be encompassed by the claims. Moreover, nothing disclosed herein is intended to be dedicated to the public regardless of whether such disclosure is explicitly recited in the claims. No claim element is to be construed as a means plus function unless the element is expressly recited using the phrase "means for."

Herein, the recitation of numerical ranges by endpoints (e.g. between about 50:1 and 1:1, between about 100 and 500 °C, between about 1 minute and 60 minutes) include all numbers subsumed within that range, for example, between about 1 minute and 60 minutes includes 21, 22, 23, and 24 minutes as endpoints within the specified range. Thus, for example, ranges 22-36, 25-32, 23-29, etc. are also ranges with endpoints subsumed within the range 1-60 depending on the starting materials used, temperature, specific applications, specific embodiments, or limitations of the claims if needed. The Examples and methods disclosed herein demonstrate the recited ranges subsume every point within the ranges because different synthetic products result from changing one or more reaction parameters. Further, the methods and Examples disclosed herein describe various aspects of the disclosed ranges and the effects if the ranges are changed individually or in combination with other recited ranges.

Further, the word "example" is used herein to mean "serving as an example, instance, or illustration." Any aspect described herein as "example" is not necessarily to be construed as preferred or advantageous over other aspects. Unless specifically stated otherwise, the term "some" refers to one or more. Combinations such as "at least one of A, B, or C," "at least one of A, B, and C," and "A, B, C, or any combination thereof' include any combination of A, B, and/or C, and may include multiples of A, multiples of B, or multiples of C. Specifically, combinations such as "at least one of A, B, or C," "at least one of A, B, and C," and "A, B, C, or any combination thereof' may be A only, B only, C only, A and B, A and C, B and C, or A and B and C, where any such combinations may contain one or more member or members of A, B, or C. Nothing disclosed herein is intended to be dedicated to the public regardless of whether such disclosure is explicitly recited in the claims.

As used herein, the term "about" and "approximately" are defined to being close to as understood by one of ordinary skill in the art. In one non-limiting embodiment, the term "about" and "approximately" are defined to be within 10%, preferably within 5%, more preferably within 1%, and most preferably within 0.5%.

### EXAMPLES

### EXAMPLE I: Preparation of Cosmetic Composition

A mixer equipped with a heating system was provided with an amount of water that equaled 5% v/v of the total water to be present in the final bulk product and heated to a temperature of 75+/-2°C. The mixer was maintained at this temperature for the entirety of the process.

Phase A raw materials were added to the mixer, which was set at a blade speed of 22 rpm with a counter blade speed of 28 rpm, a turbine speed of 1500 rpm, and a vacuum pressure of 500-600 mbar. The components were mixed for 20 minutes until dispersed completely and a gel was formed.

Next, a premix of the Phase B raw materials was added to the mixer and mixed with a blade speed of 22 rpm with a counter blade speed of 28 rpm, a turbine speed of 1500 rpm, and a vacuum pressure of 500-600 mbar. The components were mixed for 10 minutes until dispersed completely.

Next, a premix of the Phase C raw materials was added to the mixer and mixed with a blade speed of 22 rpm with a counter blade speed of 28 rpm, a turbine speed of 1500 rpm, and a vacuum pressure of 500-600 mbar for 10 minutes until dispersed completely.

Next, a premix of the Phase D raw materials was added to the mixer and mixed with a blade speed of 22 rpm with a counter blade speed of 28 rpm, a turbine speed of 1500 rpm, and a vacuum pressure of 500-600 mbar for 10 minutes until dispersed completely. The pH of the resulting homogenous mixture (that is, the homogeneous mixture of Phase A, B, C, and D) was determined. The pH was confirmed to be between 8.5 and 9.5

After the pH was confirmed to be in an acceptable range, a premix of the Phase E raw materials was added to the mixer. Prior to mixing, the pH of a sample of the components (that is, the combination of Phase A, B, C, D, and E) was determined. The pH was confirmed to be between 6 and 7.

After the pH was confirmed to be in an acceptable range, the components were mixed with a blade speed of 22 rpm with a counter blade speed of 28 rpm, a turbine speed of 1500 rpm, and a vacuum pressure of 500-600 mbar for 30 minutes until dispersed completely to provide the final bulk product.

The final bulk product was cooled to a temperature of 58-64°C and unloaded from the mixer, where aliquots were provided in multilayer LDPE bags and closed with bands, avoiding the incorporation of air in the bags. The bags were placed in a bin having an hermetic closure.

The compositions of the phases used in the process are shown in Table 1 below.

**Table 1: Phase Composition**

| **Phase** | **Components** |
|---|---|
| A | Water, Carrageenan, Propanediol |
| B | Sodium Chloride, Sodium Gluconate |
| C | pH adjusters |
| D | Pentylene Glycol, Caprylyl Glycol, Decylene Glycol |
| E | Colorant (e.g., Mica minerals) (particle size 1-5000 µm) |

## Claims

1. A method of making a cosmetic composition comprising:
providing a solvent, at least one gelling agent, at least one dispersant, at least one salt, at least one pH adjuster, and at least one preservative in a mixer to provide a first combination,
homogenizing the first combination to provide a first homogenous mixture,
performing a first pH monitoring step to determine if a pH of the first homogenous mixture is within a first specified pH range,
optionally modifying the pH of the first homogenous mixture to be within the first specified pH range,
combining the first homogenous mixture with at least one colorant to provide a second combination,
performing a second pH monitoring step to determine if a pH of the second combination is within a second specified pH range,
optionally modifying the pH of the second combination to be within the second specified pH range,
homogenizing the second combination to provide a second homogenous mixture, and
unloading the second homogenous mixture from the mixer at an elevated temperature to provide the cosmetic composition.

2. A cosmetic composition provided by a method comprising:
providing a solvent, at least one gelling agent, at least one dispersant, at least one salt, at least one pH adjuster, and at least one preservative in a mixer to provide a first combination,
homogenizing the first combination to provide a first homogenous mixture,
performing a first pH monitoring step to determine if a pH of the first homogenous mixture is within a first specified pH range,
optionally modifying the pH of the first homogenous mixture to be within the first specified pH range,
combining the first homogenous mixture with at least one colorant to provide a second combination,
performing a second pH monitoring step to determine if a pH of the second combination is within a second specified pH range,
optionally modifying the pH of the second combination to be within the second specified pH range,
homogenizing the second combination to provide a second homogenous mixture, and
unloading the second homogenous mixture from the mixer at an elevated temperature to provide the cosmetic composition.

3. The method of claim 1 or the composition of claim 2, wherein the solvent comprises water.

4. The method of claim 1 or 3 or the composition of claim 2 or 3, wherein the at least one gelling agent comprises carrageenan.

5. The method of claim 1, 3 or 4 or the composition of any one of claims 2 to 4, wherein the at least one dispersant comprises propanediol.

6. The method of any one of claims 1 or 3 to 5 or the composition of any one of claims 2 to 5, wherein the at least one preservative comprises pentylene glycol, caprylyl glycol, decylene glycol, or a combination thereof.

7. The method of any one of claims 1 or 3 to 6 or the composition of any one of claims 2 to 6, wherein the at least one colorant comprises a borosilicate, a synthetic fluorphlogopite, one or more mica minerals, or a combination thereof,
preferably wherein each of the one or more of the colorants is provided as particles with an average particle size between about 1 and 5,000 µm.

8. The method of any one of claims 1 or 3 to 7 or the composition of any one of claims 2 to 7, wherein the first specified pH range is between about 8.5 and 9.5.

9. The method of any one of claims 1 or 3 to 8 or the composition of any one of claims 2 to 8, wherein the second specified pH range is between about 6 and 7.

10. The method of any one of claims 1 or 3 to 9 or the composition of any one of claims 2 to 9, wherein at least a portion of the method is performed in a reduced pressure environment,
preferably wherein the reduced pressure environment has a pressure between about 300 and 700 mbar.

11. The method of any one of claims 1 or 3 to 10 or the composition of any one of claims 2 to 10, wherein the elevated temperature is between about 58 and 64°C.

12. The method of any one of claims 1 or 3 to 11 or the composition of any one of claims 2 to 11, wherein at least a portion of the method is performed at a temperature between about 73 and 77°C.

13. The method of any one of claims 1 or 3 to 12 or the composition of any one of claims 2 to 12, wherein the cosmetic composition is an eye shadow.

14. The method of any one of claims 1 or 3 to 13 or the composition of any one of claims 2 to 13, wherein the cosmetic composition is free of menthol.
